# EUROPÄISCHE PATENTANMELDUNG

(11) **EP 1 967 166 A1**
(43) Veröffentlichungstag der Anmeldung: **10.09.2008**
(21) Anmeldenummer: 08004207.0
(22) Anmeldetag: 06.03.2008
(51) Int. Cl.: A61F 2/44

(54) **Zwischenwirbelprothese**

(30) Priorität: 07.03.2007 DE 202007003420 U
(71) Anmelder: Brehm, Peter, 91085 Weisendorf (DE)
(72) Erfinder: Brehm, Peter, 91085 Weisendorf (DE); Gluch, Herbert, 83026 Rosenheim (DE)
(74) Vertreter: Grünecker, Kinkeldey, Stockmair & Schwanhäusser Anwaltssozietät

(57) **Zusammenfassung**

Die Erfindung betrifft eine Zwischenwirbelprothese zur Verwendung als Bandscheibenersatz im Skelett des menschlichen Körpers. Die Zwischenwirbelprothese besteht aus einer ersten Prothesenplatte (10), auf der eine ein erstes Kopplungselement z. B. in Form einer Kugelkalotte (51) angeformt ist, und einer zweiten Prothesenplatte (20), in der beispielsweise eine Hohlkugelschale (54) angeformt ist, in die im gekoppelten Zustand die Kugelkalotte eingreift. Zur Schaffung eines physiologisch günstigen Bandscheibenersatzes ist die Rotationsbewegung der beiden Prothesenplatten zueinander um eine zu den Prothesenplatten senkrechte Achse deutlich eingeschränkt, beispielsweise auf ca. 3 Grad. Die Einschränkung der Rotationsbewegung wird durch Bewegungsbegrenzungsmittel in Form mechanischer Anschläge (52,53) an den Kopplungselementen geschaffen.

## Beschreibung

Die Erfindung betrifft eine Zwischenwirbelprothese zur Verwendung als Bandscheibenersatz im Skelett des menschlichen Körpers, mit einer ersten Prothesenplatte und einer zweiten Prothesenplatte, wobei auf der ersten und zweiten Prothesenplatte jeweils Kopplungselemente mit zueinander konjugierten Flächenelementen ausgebildet sind, wobei die Kopplungselemente im gekoppelten Zustand eine Rotation sowie eine Neigungsbewegung in lateraler wie auch in posteriorer und anteriorer Richtung in Bezug auf eine zur Ausdehnung der ersten und zweiten Platte im Wesentlichen senkrecht verlaufenden Achse erlauben.

Eine Zwischenwirbel- oder auch Bandscheibenprothese ist z. B. aus der EP 1 642 553 A1 bekannt. Die dort beschriebene Bandscheibenprothese besteht aus einer ersten Prothesenplatte und einer zweiten Prothesenplatte, sowie einem zwischen der ersten Prothesenplatte und der zweiten Prothesenplatte angeordneten und getrennt davon ausgebildeten Prothesenkem. Eine der Prothesenplatten weist eine kugelschalenförmige Vertiefung auf, in die ein Kugelsegment des Prothesenkems eingreift. Die zweite Prothesenplatte weist eine im Wesentlichen ebene Vertiefung auf, in die ein dem Kugelsegment gegenüberliegender im wesentlichen flacher Abschnitt des Prothesenkems eingreift, wobei der flache Abschnitt des Prothesenkems in einer Vertiefung der zweiten Prothesenplatte verschiebbar ist. Der Prothesenkem kann nach dem Anbringen der ersten und zweiten Prothesenplatte an den jeweiligen an den Zwischenwirbelbereich angrenzenden Wirbelflächen separat eingefügt werden. Bei Neigung der Prothesenplatten zueinander ist eine laterale Verschiebung des Prothesenkems in der Vertiefung der zweiten Prothesenplatte möglich. Aufgrund der kugelförmigen Ausbildung des Prothesenkems ist eine uneingeschränkte Rotationsbewegung der ersten Prothesenplatte zur zweiten Prothesenplatte um eine im Wesentlichen zu den Flächen der Prothesenplatte senkrecht stehenden Drehachse möglich.

Körperimplantate, die als Ersatzelemente für defekte oder pathogene körpereigene Elemente oder Organe dienen, sollten die den Austauschelementen oder -organen inhärenten Eigenschaften physiologisch möglichst exakt nachbilden. Bei einem Zwischenwirbelimplantat, das als Bandscheibenersatz dienen soll, ist daher die Funktion und Ausbildung der Bandscheibe auf möglichst naturgetreue Art und Weise nachzubilden, so dass durch das Implantat die körperinhärenten Funktionen optimal nachgebildet und durch das Implantat keine unphysiologischen Prozesse initiiert werden, die in der Regel dem Wohlbefinden des Patienten abträglich sind.

Die Aufgabe der Erfindung besteht folglich darin, eine Zwischenwirbelprothese bereitzustellen, die der physiologischen Funktion und Beschaffenheit einer Bandscheibe möglichst nahe kommt und keine unphysiologischen Prozesse zur Vermeidung von durch das Implantat induzierten neuen Beschwerden schafft.

Erfindungsgemäß wird diese Aufgabe gelöst durch eine Zwischenwirbelprothese zur Verwendung als Bandscheibenersatz im Skelett des menschlichen Körpers, mit einer ersten Prothesenplatte und einer zweiten Prothesenplatte, wobei auf der ersten und zweiten Prothesenplatte jeweils Kopplungselemente mit zueinander konjugierten Flächenelementen ausgebildet sind, wobei die Kopplungselemente im gekoppelten Zustand eine Rotation sowie eine Neigungsbewegung in lateraler wie auch in posteriorer und anteriorer Richtung in Bezug auf eine zur Ausdehnung der ersten und zweiten Platte im Wesentlichen senkrecht verlaufenden Achse erlauben, wobei die Zwischenwirbelprothese dadurch gekennzeichnet ist, dass Bewegungsbegrenzungsmittel vorhanden sind, die die Rotationsbewegung auf einen vorbestimmten maximalen Wert begrenzen.

Durch die Bewegungsbegrenzungsmittel zur Begrenzung der Rotationsbewegung wird ein der menschlichen Bandscheibe angenähertes Zwischenwirbelimplantat geschaffen. Durch die Einschränkung der Rotationsbewegung in der erfindungsgemäßen Zwischenwirbelprothese werden Überlastungen der Wirbelgelenke im menschlichen Skelett, die bei konventionellen Bandscheibenersatzelementen auftreten, vermieden. Damit wird eine deutliche Verbesserung der Stabilität des gesamten Bewegungssegments erreicht, und letztendlich das Wohlbefinden des mit dem erfindungsgemäßen Zwischenwirbelimplantat ausgestatteten Patienten deutlich verbessert.

In einer bevorzugten Ausführungsform ist der maximale vorbestimmte Wert für die Rotationsbewegung ein Winkel von 5 Grad, vorzugsweise von 3 Grad. Durch diese deutliche Einschränkung der Bewegungsbegrenzung in der Rotation durch das erfindungsgemäße Zwischenwirbelimplantat wird erreicht, dass bei Rumpftorsionsbewegung des mit dem Zwischenwirbelimplantat ausgestatteten Patienten die in der Wirbelsäule dabei auftretende Verwindung auf eine größere Anzahl von Zwischenwirbelbereichen verteilt wird, und nicht lediglich durch einen, in der Rotation uneingeschränkten Zwischenwirbelbereich, hauptsächlich aufgenommen wird, so dass eine Überlastung des Band-, Muskel- und Gelenkapparats in diesem Bereich vermieden werden kann.

Gemäß einer weiteren bevorzugten Ausführungsform sind die zueinander konjugiert ausgebildeten Flächenelemente der Kopplungselemente einerseits eine Kugelkalotte, die auf der ersten Prothesenplatte vorgesehen ist, sowie eine dazu passende Hohlkugelschalenfläche, die auf der zweiten Prothesenplatte angebracht ist. Durch diese Ausbildung der Kopplungselemente, die vorzugsweise integral an den jeweiligen Prothesenplatten angeformt sind, wird eine über den vorgesehenen maximalen Bewegungsbereich optimale Kopplung geschaffen, die sowohl Rotation als auch Neigungsbewegungen in allen Richtungen durch ein einziges Kopplungselement auf einfachste Weise realisiert.

Gemäß einer bevorzugten Ausführungsform werden bei dieser Ausbildung der Kopplungselemente die Bewegungsbegrenzungsmittel als stegförmiger Überstand an der Kugelkalotte, sowie als entsprechend vorbestimmte, einen maximalen Wert des Rotationswinkels erlaubende, in der Hohlkugelschalenfläche ausgebildete Ausnehmung ausgebildet. Bei Rotationsbewegung der beiden Prothesenplatten zueinander begrenzt der an der Kugelkalotte ausgebildete stegförmige Überstand die mögliche Drehbewegung im gekoppelten Zustand der beiden Prothesenplatten dadurch, dass der stegförmige Überstand in der entsprechenden dazupassenden, jedoch größer ausgebildeten Ausnehmung am Ende des erlaubten vorbestimmten maximalen Rotationsbereichs an der Ausnehmung anschlägt. Durch geeignete Wahl der Breite des stegförmigen Überstands, bzw. geeignete Wahl der Breite der Ausnehmung lässt sich in Abhängigkeit von der Größe der Kugelkalotte die maximale Auslenkung der Rotation auf einen vorbestimmten Wert begrenzen. Durch die Wahl der Ausbildung der Höhe des stegförmigen Überstands, sowie eine geeignete entsprechende Auswahl der Tiefe der in der Hohlkugelschalenfläche ausgebildeten Ausnehmung lässt sich zudem die maximale Neigung der beiden Prothesenplatten zueinander in gekoppeltem Zustand in der Richtung, in der der stegförmige Überstand in Bezug auf den Mittelpunkt der Kugelkalotte angeordnet ist, ebenfalls auf einen vorbestimmten Wert begrenzen.

In einer weiteren bevorzugten Ausführungsform sind zwei einander gegenüberliegende Überstände vorgesehen, die in zwei einander gegenüberliegende entsprechende Ausnehmungen in die Hohlkugelschalenfläche im gekoppelten Zustand eingreifen. Dadurch wird eine besonders zuverlässige mechanische Begrenzung der Bewegung erreicht, die auch dann noch mit hoher Sicherheit die Begrenzung der Rotationsbewegung gewährleistet, zusätzlich zur Rotation eine Neigungsbewegung der beiden Prothesenplatten zueinander in Form einer Beugung oder Streckung des Wirbelgelenks auftritt. Damit wird auch bei den im physiologischen Bewegungsablauf möglichen sehr komplizierten Bewegungen, die vom Zwischenwirbelbereich aufgenommen werden müssen, eine sichere Begrenzung der Bewegungsaufnahme gewährleistet und dadurch werden unphysiologische Bewegungsabläufe auf zuverlässige Weise vermieden.

In einer bevorzugten Ausführungsform sind die Bewegungsbegrenzungsmittel derart ausgebildet, dass eine Neigungsbewegung im gekoppelten Zustand auf maximale Werte begrenzt wird, die bei der Neigung in posteriorer und anteriorer Richtung jeweils einem Wert von 15 Grad entsprechen. Vorzugsweise beträgt der maximale Wert der Begrenzung der lateralen Neigungsbewegung 6 Grad.

Damit Bewegungen sowohl in lateraler als auch in posteriorer und anteriorer Richtung, bezogen auf die Körperachse bei der in das menschliche Skelett eingefügten Zwischenwirbelprothese mit dem erfindungsgemäßen einfachen Aufbau ermöglicht werden, ist die Kugelkalotte größer ausgebildet als die entsprechende Hohlkugelschalenfläche, so dass die der Prothesenplatte angeformte Kugelkalotte sich über den vorbestimmten Neigungswinkel gegen die auf der gegenüberliegenden Prothesenplatte angeformte Hohlkugelschalenfläche verkippen lässt, bevor der Rand der Hohlkugelschale gegen die gegenüberliegende Prothesenplatte anstößt. Vorzugsweise sind die Stirnflächen der Hohlkugelschale entsprechend abgeschrägt ausgebildet, so dass bei Anschlag der Stirnflächen der Hohlkugelschale ein flächiger Anschlag gegen die gegenüberliegenden Prothesenplatte erfolgt. Vorzugsweise bestimmt der stirnseitige Rand der Hohlkugelschale eine ebene Fläche. Der laterale Neigungsanschlag wird dabei durch den Rand der Hohlkugelschale bestimmt, wogegen der Neigungsanschlag in anteriorer/posteriorer Richtung durch die Höhe des stegförmigen Überstands in der entsprechenden Ausnehmung bestimmt wird. In dieser Ausgestaltung sind somit unterschiedliche maximale Neigungswinkel in lateraler wie auch in anteriorer/posteriorer Richtung möglich.

Vorzugsweise ist zwischen der ersten und zweiten Prothesenplatte noch ein elastisches Element vorgesehen, das eine Dämpfung der Bewegung der beiden Prothesenplatten zueinander bewirkt. Dabei ist es insbesondere bevorzugt, das elastische Element im Wesentlichen die Kopplungselemente umhüllend auszubilden, so dass eine Dämpfung der Neigungsbewegungen der beiden Prothesenplatten zueinander in allen Richtungen auftritt.

Das das Kopplungselement umhüllende elastische Dämpfungselement wird dabei jeweils in Bereichen, in denen durch eine Neigungsbewegung der beiden Prothesenplatten eine Verringerung des Abstands der Prothesenplatten zueinander auftritt, auf Druck beansprucht. Das elastische Element kann nur an einer Prothesenplatte befestigt sein, so dass nur eine Druck- jedoch keine Zugbelastung des elastischen Elements auftritt. Vorzugsweise ist das elastische Element jedoch an beiden Prothesenplatten befestigt, wodurch einerseits eine Druck- und Zugbelastung zur optimalen Dämpfungswirkung auftritt und zugleich auch eine Dämpfung der Rotationsbewegung dadurch erfolgt. Schließlich besteht bei dieser Ausführungsform auch die Möglichkeit, das Kopplungselement gegen die Umgebung abzudichten. Auch wenn dies aus physiologischen Gründen nicht dringend notwendig erscheint, da der Zwischenwirbelbereich weitgehend frei von Körpermaterial und -flüssigkeiten ist, so ließe sich dadurch dennoch eine noch weitergehende Verbesserung und Zuverlässigkeit des Kopplungssystems erreichen.

In einer weiteren bevorzugten Ausführungsform ist jeweils in den Bereichen, in denen bei Neigungsbewegung sowohl in lateraler als auch in posteriorer und anteriorer Richtung Elemente der ersten Prothesenplatte oder der daran angeformten Kopplungselemente gegen Bereiche der zweiten Prothesenplatte oder der daran angeformten entsprechenden Kopplungselemente bei Maximalneigung anschlagen, mit jeweiligen Anschlagsdämpfungsmitteln ausgestattet, die in vorzugshafter Weise aus einem schichtförmigen elastischen Material hergestellt sind. Dadurch werden sowohl mechanische Überbeanspruchungen des Materials der Prothesenplatten und der Kopplungselemente, die gegebenenfalls zu Beschädigung oder Absplitterungen führen können, vermieden, als auch bei Anschlag auftretende eventuelle Geräusche, die sich beim Patienten als störend erweisen könnten, ausgeschlossen.

Vorteilhafterweise werden die ersten und zweiten Prothesenplatten aus Kobaltchromlegierung, aus Titan oder aus Implantatstahl hergestellt. Durch Beschichtung der knochenzugewandten Seiten der Prohesenplatten mit Titanplasma und/oder Hydroxylapatitvergütung wird eine verbesserte knöcherne Integration der Prothesenplatten sichergestellt. Zur Reduzierung des Abriebs der Kopplungselemente lassen sich diese auch aus Keramik herstellen.

In einer weiteren bevorzugten Ausführungsform sind die Prothesenplatten derart gefertigt, dass an den jeweils den Kopplungselementen abgewandten Seiten eine konvexe Form oder Krümmung vorgesehen ist. Damit lässt sich das Zwischenwirbelimplantat operationstechnisch besonders einfach in den Zwischenwirbelspalt einfügen. Im Vergleich zu den bei Zwischenwirbelimplantaten nach dem Stand der Technik meist sehr weit vorstehenden Pins oder Graten, die tief in den jeweils benachbarten Wirbelkörper eingreifen, schafft die nur als konvexe Form oder Krümmung ausgebildete Verankerung der Prothesenplatte an dem Wirbelkörper zwar immer noch eine ausreichende Sicherheit der Befestigung der Prothesenplatte im Skelett, ermöglicht jedoch einen wesentlichen leichteren Einbau und gestattet auch eine Revisionsoperation, bei der das Zwischenwirbelimplantat entnommen und durch ein Ersatzprodukt ersetzt werden kann. In einer besonders bevorzugt Ausführungsform ist die konvex geformte Oberfläche der jeweiligen Prothesenplatte an die innere Kontur des Wirbelkörpers im Zwischenwirbelbereich angepasst. Operationstechnisch lässt sich das Zwischenwirbelimplantat gemäß der Erfindung somit sehr einfach einfügen, indem die Wirbelsäule des Patienten im Operationsverlauf im entsprechenden Bereich stark gekrümmt wird, wodurch sich in dem relevanten Zwischenwirbelbereich ein sich nach außen öffnender Spalt bildet, in den sich das erfindungsgemäße Zwischenwirbelimplantat mit der konvex gestalteten Außenoberfläche der jeweiligen Prothesenplatte für den Operateur leicht einfügen lässt, wogegen Prothesenplatten nach dem Stand der Technik einzeln eingefügt werden mussten, und die Kopplungselemente anschließend separat zwischen die beiden bereits in dem Wirbelkörper verankerten Prothesenplatten anschließend eingebaut werden mussten. Die erfindungsgemäße Ausgestaltung der Prothesenplatten in dieser Ausführungsform erlaubt daher eine einstückige Anformung der Kopplungselemente an die jeweiligen Prothesenplatten, so dass dadurch auch wiederum eine Vereinfachung des Zwischenwirbelimplantats möglich ist, was zu einer Verbesserung der Zuverlässigkeit des Implantats und zu einer Verringerung in den Herstellungskosten des Implantats beiträgt.

Gemäss einer weiteren bevorzugten Ausführungsform sind die zueinander konjugierten Flächenelemente der Kopplungselemente geeignet ausgebildet, dass bei einer Neigung der ersten und zweiten Prothesenplatte zueinander zugleich eine entsprechende translatorische Bewegung auftritt. Insbesondere beinhaltet diese Ausbildung der konjugierten Flächenelemente der Kopplungselemente die Verwendung einer Kugelkalotte, die nur teilweise in einer entsprechenden Hohlkugelschalenfläche aufgenommen ist. Bei Neigung der beiden Prothesenplatten zueinander wandert dabei die Hohlkugelschalenfläche über der Kugelkalotte entlang der Oberfläche der Kugelkalotte, wobei zugleich eine gewisse translatorische Bewegung der an der Hohlkugelschalenoberfläche angeformten Prothesenplatte gegenüber der gegenüberliegenden Prothesenplatte unter gleichzeitiger Verkippung auftritt.

Die translatorische Bewegung lässt sich durch die Wahl des Radius der Kugelkalotte auf das physiologisch geeignete Maß einstellen.,Vorzugsweise ist der Radius der Kugelkalotte das 0,5- bis 0,8-fache der anterioren/posterioren Erstreckung der ersten Prothesenplatte.

Schließlich ist in einer weiteren vorteilhaften Ausführungsform eine Auskleidung zwischen der Kugelkalotte und dem Sitz in der Hohlkugelschalenfläche vorgesehen, die wiederum aus einem dünnen schichtförmigen elastischen Material bestehen kann.

Die vorgenannten vorzugsweise beschriebenen Merkmale können jeweils einzeln oder in beliebiger Kombination miteinander in dem erfindungsgemäßen Zwischenwirbelimplantat, wie es in Patentanspruch 1 spezifiziert ist, vorgesehen sein.

Nachfolgend wird die Erfindung anhand von Ausführungsbeispielen näher erläutert und beschrieben. In den die Erfindung beispielhaft veranschaulichenden Zeichnungen zeigen:
- Fig. 1: eine dreidimensionale Gesamtansicht eines erfindungsgemäßen Zwischenwirbelimplantats,
- Fig. 2: eine Sprengdarstellung des in der Fig. 1 gezeigten Zwischenwirbelimplantats,
- Fig. 3: das erfindungsgemäße Zwischenwirbelimplantat in aufgeklapptem Zustand,
- Fig. 4: eine Querschnittsansicht entlang der Linie IV-IV von Fig. 1,
- Fig. 5: eine Querschnittsansicht entlang der Linie V-V von Fig. 1 bei anteriorer Verkippung,
- Fig. 6: eine vergrößerte Darstellung des Teilbereichs X in Fig. 4,
- Fig. 7: eine Seitenansicht aus anteriorer Richtung des erfindungsgemäßen Zwischenwirbelimplantats, sowie
- Fig. 8: die Seitenansicht aus posteriorer Richtung bei Verkippung und Rotation des Zwischenwirbelimplantats.

Nachfolgend soll die Erfindung anhand der vorstehend bezeichneten Figuren näher erläutert und beschrieben werden. Einleitend sei zunächst der physiologische Hintergrund für das Zwischenwirbelimplantat erläutert.

Das erfindungsgemäße Zwischenwirbelimplantat dient als Ersatz für eine Bandscheibe. Die körpereigene Bandscheibe stellt ein Gebilde mit einem Gallertkem und einem Faserring dar, die zwischen zwei Endplatten vorhanden sind. Die Bandscheibe liegt zwischen zwei Wirbelkörpern und ist damit ein Teil eines Bewegungssegments, das physiologisch als Grundelement des Bewegungs- und Stabilitätssystems der Wirbelsäule wirkt. Die Bandscheibe, sofern sie intakt ist, besitzt die Funktion einer Stabilisierung der Wirbelsäule, einer Kontrolle der Beweglichkeit des Bewegungssegments, was durch den besonderen Aufbau des Faserrings hervorgerufen wird, einer Abstandshaltung zwischen den Wirbelkörpern, wobei diese Funktion auch die Kontrolle der Beweglichkeit bewirkt, und einer Dämpfung bei axialen Belastungen der Wirbelsäule. Die natürliche Bandscheibe vermittelt somit zwischen zwei Wirbelkörpern eine eingeschränkte und gedämpfte Relativbewegung in Bezug auf Verkippung wie auch axiale Rotation. Die Beweglichkeit des Bewegungssystems ändert sich jedoch im Laufe des Lebens. Während die Beugung, Streckung und die Seitenbewegung im Laufe einer Bandscheibendegeneration eingeschränkt wird, nimmt die Rotationsbewegung im Laufe des Degenerationsprozesses der Bandscheibe zu.

Bei der Entwicklung der vorliegenden Erfindung wurde erkannt, dass insbesondere die Rotationsbewegung besondere Bedeutung für die anatomisch und physiologisch korrekte Beweglichkeit des Bewegungssystems besitzt. Die physiologische Kontrolle der Bewegungsmöglichkeiten des Bewegungssegments in Kombination aus Bandscheiben- und Wirbelgelenk und Bandapparat sowie der Muskulatur, ist daher auch im Hinblick auf ein künstliches Implantat einer Zwischenwirbelprothese notwendig.

Die aus dem Stand der Technik bekannten Zwischenwirbelprothesen schaffen noch keine ausreichende Kontrolle über das Bewegungssegment in der Wirbelsäule. Insbesondere besteht die Problematik, dass die vorhandenen Zwischenwirbelimplantate zuviel Beweglichkeit erlauben und somit eine Überlastung der eventuell aufgrund der Operation bereits verringerten Anzahl und möglicherweise bereits durch Verschleiß veränderten Bänder verursachen.

Der erfindungsgemäße Ansatz besteht nun darin, eine Zwischenwirbelprothese als Bandscheibenersatz im Skelett des menschlichen Körpers bereitzustellen, bei dem eine kontrollierte Beweglichkeit zur Schonung des Bandsystems sowohl des betroffenen Zwischenwirbelbereichs als auch der bei physiologischen Bewegungsabläufen beteiligten Band-, Skelett- und Muskelsystemen geschaffen wird. Daher ist die Kontrolle der Beweglichkeit bei der Konstruktion eines Bandscheibenersatzes von großer Wichtigkeit, insbesondere da eine Zwischenwirbelprothese als Bandscheibenersatz bei degenerativen Veränderungen verwendet wird. Somit wird durch die erfindungsgemäße Zwischenwirbelprothese als Bandscheibenersatz ein deutlich verbessertes Implantat geschaffen, da eine deutlich eingeschränkte Drehbeweglichkeit in dem mit dem erfindungsgemäßen Zwischenwirbelimplantat versehenen Bewegungssegment geschaffen wird. Dies führt zu einer Entlastung der beteiligten Bänder und einer Unterstützung der Wirbelgelenke, welche bei aus dem Stand der Technik bekannten Modellen mit vollständiger Rotationsbeweglichkeit deutlich überfordert waren. Dies führt letztendlich zu einer besseren dauerhaften Stabilität des gesamten Bewegungssegments. Die erfindungsgemäße Zwischenwirbelprothese als Bandscheibenersatz besitzt die Fähigkeit, am Zwischenspiel mit den Wirbelgelenken teilzunehmen, um einen harmonischen Bewegungsablauf zu sichern. Die erfindungsgemäße Zwischenwirbelprothese schafft sowohl den physiologischen Bewegungsablauf als auch eine Dämpfungsfunktion axialer Bewegungen in der Wirbelsäure unter zusätzlicher Verwendung eines Dämpfungsinlays in dem Kopplungssystem. Durch die Ausbildung des Kopplungselements als eine Kugelkalotte, die in eine Hohlkugelschalenfläche ohne vollständige Umgreifung der Kugelkalotte eingreift, wird neben einer Kipp-/Neigungsbewegung auch eine translatorische Bewegung geschaffen.

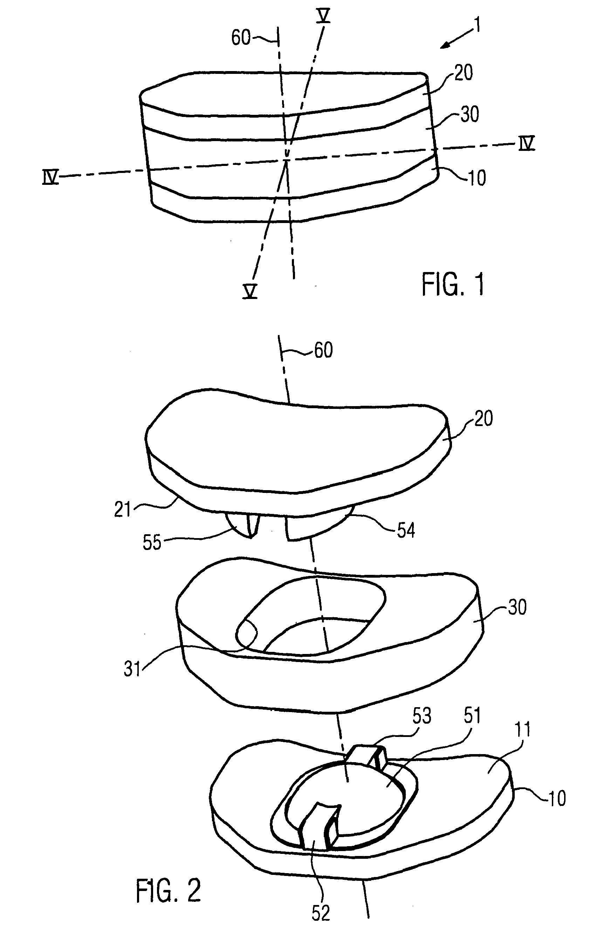

In der Fig. 1 ist die erfindungsgemäße Zwischenwirbelprothese im zusammengesetzten Zustand gezeigt. Die Zwischenwirbelprothese 1 umfasst eine erste Prothesenplatte 10 und eine zweite Prothesenplatte 20. Zwischen der ersten und zweiten Prothesenplatte ist ein elastisches Dämpfungselement 30 vorgesehen. Im nicht gekippten Zustand sind die erste Prothesenschale 10, die zweite Prothesenschale 20 und das elastische Dämpfungselement 30 im gekoppelten Zustand spaltfrei zusammengefügt. Das elastische Dämpfungselement 30 bildet mit den Seitenflächen der ersten und zweiten Prothesenplatte 10 und 20 jeweils im Wesentlichen glatt übergehende Abschlusskanten.

In der Sprengdarstellung der Fig. 2 ist ein erster Teil eines an die Prothesenplatte 10 angeformten Kopplungselements gezeigt. Dieser Teil des Kopplungselements umfasst eine Kugelkalotte 51, an der zwei seitliche stegartige Überstände 52 und 53 ausgebildet sind. An der zweiten Prothesenplatte 20 ist ein weiterer Teil des Kopplungselements angeformt, der eine Hohlkugelschale 54 aufweist, in der Ausnehmungen 55 und 56 zur Aufnahme der stegförmigen Überstände 52 und 53 ausgebildet sind. Die Ausnehmungen 55, 56 sind von der Größe so an die stegförmigen Überstände 52 und 53 angepasst, dass im gekoppelten

Zustand die stegförmigen Überstände 52 und 53 Spiel aufweisen, das eine Rotationsverdrehung einer Prothesenplatte gegenüber der anderen um einen vorbestimmten Winkel erlaubt, bis die Seitenfläche eines stegartigen Überstands an der Innenseitenfläche der entsprechenden Ausnehmung anstößt. Die Ausnehmungen sind im Vergleich zu der Größe der stegartigen Überstände so gestaltet, dass im gekoppelten Zustand der ersten und zweiten Prothesenplatte eine maximale Rotationsbewegung von 3 Grad um eine zur Fläche der Prothesenplatte 10 oder 20 senkrecht verlaufende Achse möglich ist. Die äussere feie Fläche der Kugelkalotte 51 ist größer bemessen als die entsprechende Aushöhlung, die durch die Hohlkugelschale 54 geschaffen wird.

Das elastische Dämpfungselement 30 weist eine im Wesentlichen gleiche Dicke über sein seitliche Erstreckung auf und besitzt in einem mittleren Bereich eine Ausnehmung 31, die eine Aufnahme der an der ersten und zweiten Prothesenplatte angeformten Kupplungselemente ermöglicht. Die Dicke des elastischen Dämpfungselements 30 ist derart gewählt, dass bei zueinander parallelen ersten und zweiten Prothesenplatten das Dämpfungselement spaltfrei an den Innenflächen 11 und 21 der ersten und zweiten Grundplatte 10 und 20 anliegt.

Das elastische Dämpfungselement kann durch spezielle Formgebung, z. B. einknöpfbare Noppen, in der ersten und/oder zweiten Prothesenplatte befestigt sein. Eine Befestigung des elastischen Dämpfungselements an der ersten und/oder zweiten Prothesenplatte kann auch durch geeignete Vorsprünge auf den Innenfläche 11 und 21 der ersten und zweiten Prothesenplatte geschaffen werden, z. B. durch schwalbenschwanzförmige Ausbildungen, pilzförmige Ausbildungen oder Ähnliches, die in entsprechend geeignete Vertiefungen in dem elastischen Element fixierbar sind (in den Figuren nicht gezeigt).

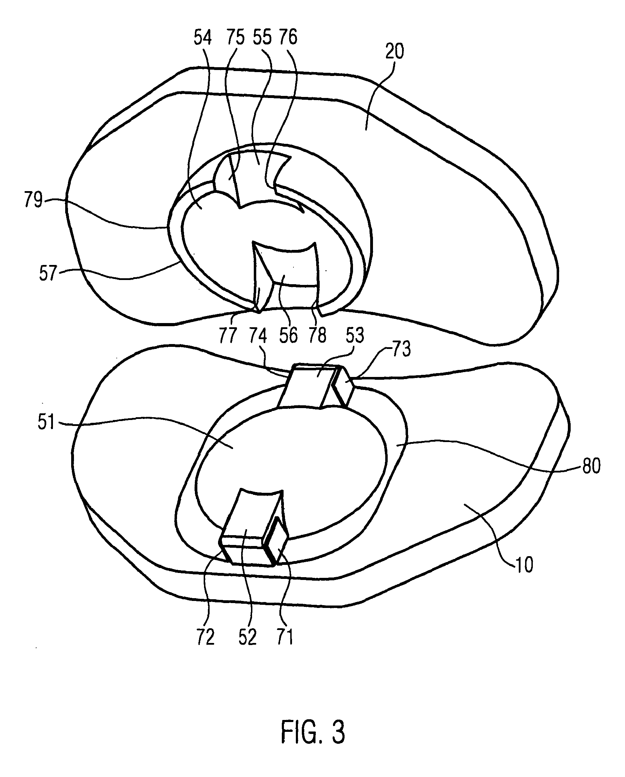

In der Darstellung der Fig. 3 sind zusätzliche Dämpfungselemente im Bereich der Anschlagflächen der Bewegungsbegrenzungsmittel gezeigt. An der ersten Prothesenplatte 10 ist an den Seitenflächen der stegförmigen Überstände 52 und 53 jeweils flächige elastische Dämpfungsauflagen 71 und 72, sowie 73 und 74 vorgesehen, die zur Begrenzung der Rotationsbewegung als Rotationsanschlag dienen. In gleicher Weise kann in den Seiteninnenflächen der Ausnehmungen 55 und 56 der an der zweiten Prothesenplatte 20 ausgebildeten Hohlkugelschale 54 jeweils ein flächiger elastischer Dämpfungsbelag 75, 76, 77, 78 vorgesehen sein. Am stirnseitigen Rand 57 der Hohlkugelschale 54 ist weiter eine flächige elastische Dämpfungsauflage 79 vorgesehen. Zusätzlich kann auf der ersten Prothesenplatte 10 seitlich an die Kugelkalotte 51 anschließend eine flächige elastische Dämpfungsauflage 80 vorhanden sein. Die elastischen Dämpfungsauflagen 79 und 80 dämpfen die Anschlagflächen beim Neigungsanschlag elastisch ab.

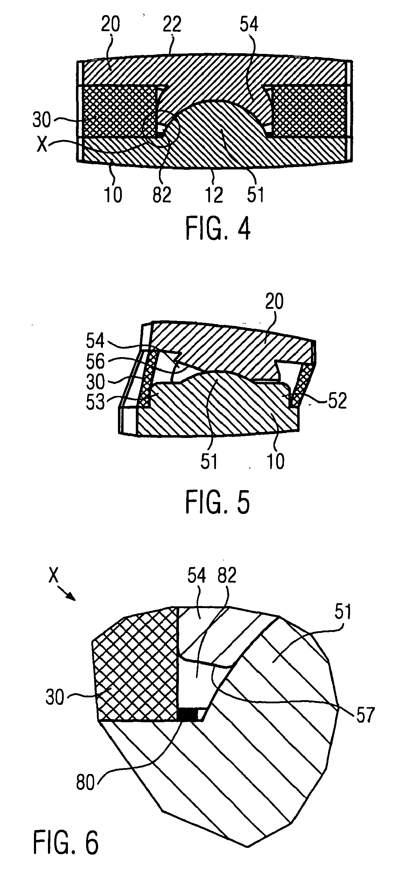

In der Querschnittsansicht der Fig. 4 wird veranschaulicht, dass die Hohlkugelschale 54 die Kugelkalotte 51 nur teilweise aufnimmt, so dass ein Spalt zwischen der stirnseitigen Randfläche 57 der Hohlkugelschale und der inneren Fläche 11 der ersten Prothesenplatte verbleibt. Aufgrund dieses Spalts ist eine Neigung der ersten Prothesenplatte 10 zur zweiten Prothesenplatte 20 um einen vorbestimmten Wert möglich, der in einer bevorzugten Ausführungsform auf einen Maximalwert von 10 Grad in lateraler Richtung begrenzt ist.

In der Fig. 5 ist die erfindungsgemäße Zwischenwirbelprothese in einem seitlichen Schnitt gezeigt, der die translatorische Verschiebung und Neigung der ersten und zweiten Prothesenplatte zueinander bei anteriorer/posteriorer Beugung veranschaulicht. Wie bereits in der Fig. 2 zu erkennen war, ist in der Schnittansicht der Fig. 5 nochmals die wesentlich dünnere Ausgestaltung der durch die Ausnehmung 31 in dem elastischen Dämpfungselement 30 gebildeten Wände in der anterioren und posterioren Position im Vergleich zu den lateralen Wandstärken zu erkennen. Darüber hinaus ist der Bewegungsanschlag bei maximaler Neigung der ersten und zweiten Prothesenplatte 10 und 20 zueinander bei Beugung nach vorne zu erkennen. Aufgrund der auf der Kugelkalotte nach anterior (in der Fig. rechts) gleitenden Hohlkugelschale findet insgesamt auch eine Translation der zweiten Prothesenplatte gegenüber der ersten Prothesenplatte in anteriorer Richtung statt. Die innen liegenden Stirnflächen der Ausnehmung 55 und 56 in der Hohlkugelschale 54 sind so abgeschrägt, dass eine flächige Anlage auf den oben liegenden Deckflächen der stegförmigen Überstände 52 und 53 stattfindet. Durch die Auflage der innen liegenden Stirnflächen der Ausnehmungen 55 und 56 auf den oben liegenden Deckflächen der stegförmigen Überstände 52 und 53 wird die maximale Verkippung der ersten und zweiten Prothesenplatte zueinander in anteriorer und posteriorer Richtung, also Beugung nach vorne und Streckung nach hinten, bestimmt. Im gezeigten Beispiel beträgt die maximale Beugung 10 Grad. Abhängig davon, in welchen Zwischenwirbelbereich in der Wirbelsäule die Zwischenwirbelprothese eingesetzt werden soll, kann die maximale Beugung zwischen 10 und 15 Grad betragen. Die maximale laterale Beugung, die durch Auflage des Randes 57 der Hohlkugelschale and der Innefläche der ersten Prothesenplatte bestimmt wird, kann, ebenfalls abhängig von dem Einsatzort an der Wirbelsäule, vorzugsweise zwischen 5 und 6 Grad betragen.

Wie in der Fig. 4 zu erkennen ist, sind die den Kopplungselementen gegenüberliegenden Außenflächen 12 und 22 der ersten und zweiten Prothesenplatte 10 und 20 konvex ausgebildet. Durch diese Konvexität liegt ein Teil des Volumens der ersten und zweiten Prothesenplatte 10 und 20 innerhalb des Knochenrands der angrenzenden Wirbel, wenn die Zwischenwirbelprothese in das Skelett als Bandscheibenersatz eingesetzt ist. Dadurch wird eine selbstklemmende Wirkung der erfindungsgemäßen Zwischenwirbelprothese erzielt. In vorteilhafter Weise ist die konvexe Form oder Krümmung der äußeren Oberflächen 12 und 22 der ersten und zweiten Prothesenplatte an die anatomische Formgebung der Innenwirbelflächen durch eine komplementäre Innenflächentopologie angepasst (nicht gezeigt). Damit ist ein noch besserer Sitz der Prothesenplatten im Zwischenwirbelbereich gewährleistet.

In der Fig. 6 ist der in der Fig. 4 gezeigte Abschnitt X, in dem der Spalt zwischen dem Rand 57 der Hohlkugelschale und der Innerfläche 11 der ersten Prothesenplatte gezeigt ist, vergrößert dargestellt. Der stirnseitige Rand 57 der Hohlkugelschale 54 ist in Richtung zur Innenfläche der zweiten Prothesenplatte abgeschrägt, so dass bei vollständiger Beugung und Anschlag der stirnseitigen Randfläche 57 am Dämpfungselement 80 auf der Innenfläche der ersten Prothesenplatte 10 eine ganzflächige Auflage unter lateraler Verdrängung des elastischen Dämpfungselements 30 stattfindet.

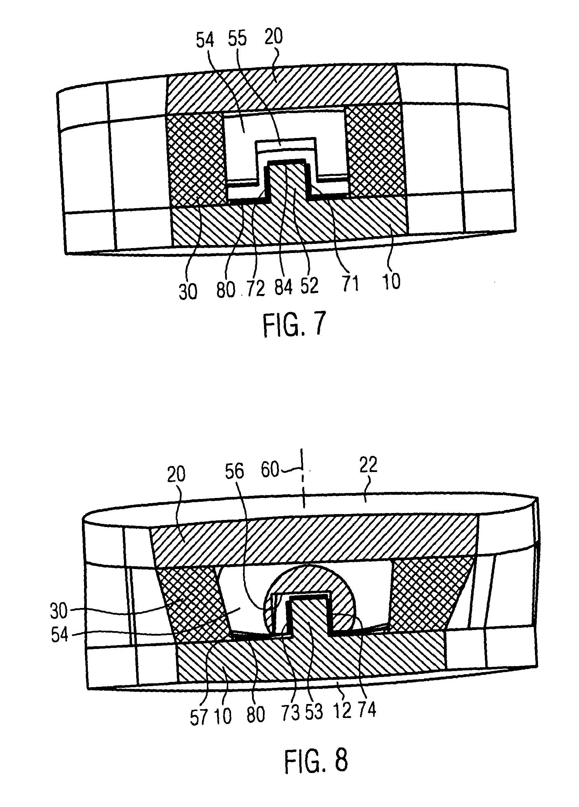

In der Fig. 7 ist eine Seitenansicht der Zwischenwirbelprothese von vorne gezeigt. In dieser Darstellung ist auf der oberen Anschlagfläche des stegförmigen Überstands 52 ein weiterer flächiger Anschlagdämpfer 84 ausgebildet. In der gezeigten Ausführungsform findet bei maximaler Neigung der beiden Prothesenplatten zueinander nach anterior ein Anschlag sowohl auf der oberen Anschlagfläche 84 des stegförmigen Überstands 52, als auch auf der inneren Oberfläche der Prothesenplatte 10 seitlich angrenzend an den stegförmigen Überstand, an dem ebenfalls Dämpfungselemente 80 vorhanden sind, statt. Der Anschlag ließe sich jedoch auch allein durch Begrenzung des Anschlags auf der oberen Stegfläche, an der das Dämpfungselement 84 vorgesehen ist, erzielen. In diesem Fall bestünde keine Einschränkung der maximal einstellbaren Anschlagwinkel in verschiedenen Richtungen, so dass sich zwischen anterior/posterior und lateral links und rechts jeweils unterschiedliche maximale Neigungswinkel einstellen ließen. Bei Verwendung der oberen Oberfläche des stegförmigen Überstands als alleinige Anschlagfläche für die Endbegrenzung des maximalen Neigungswinkels anterior und posterior ist es vorteilhaft, die obere Oberfläche des stegförmigen Überstands wie auch inner Fläche der entsprechenden Ausnehmung entsprechend gewölbt auszubilden, um weiterhin großflächige Auflageflächen im Anschlagbereich zu schaffen, wenn gleichzeitig eine laterale Verkippung auftritt.

In der Fig. 8 ist eine Seitenansicht der erfindungsgemäßen Zwischenwirbelprothese von posterior aus gezeigt. In der dargestellten Ausführungsform ist zudem die erste Prothesenplatte 10 gegenüber der zweiten Prothesenplatte 20 um den maximalen Rotationswinkel um die zu den Prothesenplatten senkrecht stehende Achse 60 verdreht. Zugleich ist die zweite Prothesenplatte 20 gegenüber der ersten Prothesenplatte 10 maximal nach posterior verkippt, so dass der stirnseitige Rand 57 der Hohlkugelschale 54 auf dem flächigen Anschlagsdämpfer 80 beidseitig des stegförmigen Überstands 53 aufliegt. Der flächige Anschlagdämpfer 74 an der Seite des stegförmigen Überstands 53 liegt an der Innenfläche der Ausnehmung 56 in der Hohlkugelschale 54 an. Das Dämpfungselement 30 ist in dieser Konstellation beidseitig der Kopplungselemente auf Druck beansprucht.

Während vorstehend eine bevorzugte Ausführungsform der Erfindung beschrieben wurde, so ist für den Fachmann ersichtlich, dass die Erfindung grundsätzlich nicht auf die gezeigte mechanische Lösung zur Bewegungsbegrenzung oder zur Kopplung der beiden Prothesenplatten begrenzt ist. Beispielsweise könnten die Stege in der Hohlkugelschale angeordnet sein, während entsprechende Ausnehmungen in der Kugelkalotte vorgesehen sind. Anstelle von paarweisen randseitigen Ausnehmungen und stegförmigen Überständen an den jeweiligen Kopplungselementen wären auch einzelne, dafür zentral an der Kugelkalotte und in der Hohlkugelschalenfläche angeordnete Ausnehmungen und stegförmige Vorsprünge möglich. Auch die bezeichnete Form der Stege und Ausnehmungen als im Wesentlichen quaderförmig ist für den Fachmann ersichtlich nicht erfindungswesentlich, sondern andere Arten der Formgebung der Stege und Ausnehmungen sind denkbar, solange eine Asymmetrie zur Begrenzung der Rotationsbewegung der beiden Prothesenplatten zueinander vorhanden ist.

Die Bewegungsbegrenzungselemente könnten auch getrennt von den Kopplungselementen an der ersten und zweiten Prothesenplatte angeformt oder ausgebildet sein.

### Bezugszeichenliste

- 1: Zwischenwirbelprothese
- 10: erste Prothesenplatte
- 11: Innenfläche der ersten Prothesenplatte
- 12, 22: äußere Oberfläche der ersten Prothesenplatte
- 20: zweite Prothesenplatte
- 21: Innenfläche der zweiten Prothesenplatte
- 22: äußere Oberfläche der zweiten Prothesenplatte
- 30: Dämpfungselement
- 51: Kugelkalotte
- 52, 53: stegartige Überstände
- 54: Hohlkugelschale
- 55, 56: Ausnehmungen in Hohlkugelschale
- 57: stirnseitiger Rand
- 60: Achse
- 71,72,73,74: Dämpfungsauflage auf stegartigen Überständen
- 75, 76, 77, 78: Dämpfungsauflage auf Seitenflächen der Ausnehmungen
- 79: elastische Dämpfungsauflage auf stirnseitgem Rand
- 80: elastische Dämpfungsauflage seitlich der Kugelkalotte
- 82: Spaltabschnitt
- 84: Anschlagdämpfer auf stegartigem Überstand

## Patentansprüche

1. Zwischenwirbelprothese (1) zur Verwendung als Bandscheibenersatz im Skelett des menschlichen Körpers, mit einer ersten Prothesenplatte (10) und einer zweiten Prothesenplatte (20), wobei auf der ersten und zweiten Prothesenplatte Kopplungselemente (51, 54) mit zueinander konjugierten Flächenelementen ausgebildet sind, wobei die Kopplungselemente im gekoppelten Zustand eine Rotation sowie eine Neigungsbewegung in lateraler wie auch in posteriorer und anteriorer Richtung in Bezug auf eine zur Ausdehnung der ersten und zweiten Platte im Wesentlichen senkrecht verlaufenden Achse (60) erlauben, **dadurch gekennzeichnet, dass** Bewegungsbegrenzungsmittel (52, 53, 55, 56) vorhanden sind, die die Rotationsbewegung auf einen vorbestimmten maximalen Wert begrenzen.

2. Zwischenwirbelprothese gemäß Anspruch 1, wobei der vorbestimmte maximale Wert ein Rotationswinkel von 5°, vorzugsweise 3°, ist.

3. Zwischenwirbelprothese gemäß Anspruch 1 oder 2, wobei die zueinander konjugiert ausgebildeten Flächenelemente der Kopplungselemente eine Kugelkalotte (51), die auf der ersten Prothesenplatte (10) vorgesehen ist, und eine dazu passende Hohlkugelschalenfläche (54), die auf der zweiten Prothesenplatte (20) angebracht ist, umfassen.

4. Zwischenwirbelprothese gemäß Anspruch 3, wobei am Basisbereich der Kugelkalotte wenigstens ein stegförmiger Überstand (52, 53) ausgebildet ist, der in eine, den entsprechenden vorbestimmten maximalen Wert des Rotationswinkels begrenzende, in der Hohlkugelschalenfläche ausgebildete Ausnehmung (55, 56) im gekoppelten Zustand eingreift.

5. Zwischenwirbelprothese gemäß Anspruch 4, wobei an der Kugelkalotte zwei aneinander gegenüberliegende Überstände (52, 53) vorgesehen sind, die in zwei einander gegenüberliegende entsprechende Ausnehmungen in der Kugelschale im gekoppelten Zustand eingreifen.

6. Zwischenwirbelprothese gemäß einem der Ansprüche 1 bis 5, wobei die Bewegungsbegrenzungsmittel derart ausgebildet sind, dass eine Begrenzung der Neigungsbewegung in posteriorer und anteriorer Richtung auf einen vorbestimmten maximalen Wert von 15° erfolgt.

7. Zwischenwirbelprothese gemäß einem der vorhergehenden Ansprüche, wobei die Kopplungselemente geeignet ausgebildet sind, im gekoppelten Zustand die Neigungsbewegung in lateraler Richtung auf einen vorbestimmten maximalen Wert zu begrenzen.

8. Zwischenwirbelprothese gemäß Anspruch 7, wobei der maximale Wert der Begrenzung der lateralen Neigungsbewegung 6° beträgt.

9. Zwischenwirbelprothese gemäß einem der vorhergehenden Ansprüche, wobei die zueinander konjugiert ausgebildeten Flächenelemente der Kopplungselemente eine Kugelkalotte (51), die auf der ersten Prothesenplatte (10) vorgesehen ist, und eine dazu passende Hohlkugelschalenfläche (54), die auf der zweiten Prothesenplatte (20) angebracht ist, umfassen, wobei die Kugelkalotte nur teilweise in der Hohlkugelschalenfläche aufgenommen wird.

10. Zwischenwirbelprothese gemäß Anspruch 9, wobei Stirnflächen (57) der Hohlkugelschale (54) jeweils lateral in Richtung zur Prothesenplatte abgeschrägt ausgebildet sind.

11. Zwischenwirbelprothese gemäß einen der vorhergehenden Ansprüche, wobei ein, vorzugsweise die Kopplungselemente im Wesentlichen umhüllendes, elastisches Element (30) zwischen der ersten und zweiten Prothesenplatte vorgesehen ist.

12. Zwischenwirbelprothese gemäß Anspruch 11, wobei das elastische Element (30) an wenigstens einer der ersten und zweiten Prothesenplatte befestigt ist.

13. Zwischenwirbelprothese gemäß einem der Ansprüche 1 bis 12, wobei Anschlagdämpfungsmittel (71 - 78) im Bereich des Anschlags der Rotations- und/oder Neigungsbewegung vorgesehen sind.

14. Zwischenwirbelprothese gemäß Anspruch 13, wobei die Anschlagsdämpfungsmittel aus einem schichtförmigen elastischen Material hergestellt sind.

15. Zwischenwirbelprothese gemäß einem der Ansprüche 1 bis 14, wobei die erste und zweite Prothesenplatte aus Kobaltchromlegierung hergestellt sind.

16. Zwischenwirbelprothese gemäß einem der Ansprüche 1 bis 14, wobei die erste und zweite Prothesenplatte aus Titan hergestellt sind.

17. Zwischenwirbelprothese gemäß einem der Ansprüche 1 bis 14, wobei die erste und zweite Prothesenplatte aus Keramik hergestellt sind.

18. Zwischenwirbelprothese gemäß einem der vorhergehenden Ansprüche, wobei die zueinander konjugierten Flächenelemente wenigstens teilweise mit Titanplasma und/oder Hydroxylapatit beschichtet sind.

19. Zwischenwirbelprothese gemäß einem der Ansprüche 1 bis 18, wobei wenigstens eine der ersten und zweiten Prothesenplatte auf der den Kopplungselementen abgewandten Seite (12, 22) konvex ausgebildet ist.

20. Zwischenwirbelprothese gemäß Anspruch 19, wobei die erste und zweite Prothesenplatte auf der den Kopplungselementen abgewandten Seiten (12, 22) eine den Zwischenwirbelflächen der Wirbel nachgestaltete, dazu komplementäre Flächentopologie aufweisen.

21. Zwischenwirbelprothese nach einem der vorhergehenden Ansprüche, wobei die zueinander konjugierten Flächenelemente der Kopplungselemente geeignet ausgebildet sind, eine translatorische Bewegung der ersten und zweiten Prothesenplatte in posteriorer/anteriorer Richtung im gekoppelten Zustand zu erlauben.

22. Zwischenwirbelprothese gemäß einem der vorhergehenden Ansprüche, wobei die beien Teile der Kopplungselemente jeweils integral mit der ersten und zweiten Prothesenplatte ausgebildet sind.
